# EUROPEAN PATENT APPLICATION

(11) **EP 0 533 952 A1**
(43) Date of publication of application: **31.03.1993**
(21) Application number: 92908243.6
(22) Date of filing: 15.04.1992
(51) Int. Cl.: C12Q 1/68, C12N 15/10

(54) **PROCESS FOR SEPARATING OR EXTRACTING NUCLEIC ACID FROM SPECIMEN CONTAINING THE SAME**

(30) Priority: 15.04.1991 JP 108187/91; 24.04.1991 JP 122479/91
(71) Applicant: SUMITOMO ELECTRIC INDUSTRIES, LTD., Osaka-shi, Osaka 541 (JP)
(72) Inventor: KISHIMOTO, Toshihiko, Osaka Works of Sumitomo, Konohana-ku, Osaka-shi, Osaka 554 4 (JP); NIWA, Shin-ichiro, Osaka Works of Sumitomo, Konohana-ku, Osaka-shi, Osaka 554 54 (JP); TSURUI, Hiromichi, Nerima-ku, Tokyo 177 (JP)
(74) Representative: Hansen, Bernd, Dr.rer.nat.
(86) International application number: JP9200473
(87) International publication number: WO9218647

(57) **Abstract**

A process for separating or extructing a specified nucleic acid from the solution of a specimen containing nucleic acids which comprises the steps of: (1) immobilizing a single-stranded nucleic acid on an insoluble carrier at its terminal, (2) contacting the immobilized nucleic acid with the specimen containing nucleic acids in solution under the nucleic acid hybridization condition, and (3) isolating the nucleic acid which hybridizes with the immobilized nucleic acid from the specimen. This process can realize simultaneous separation of two or more single-stranded nucleic acids which are different from one another in the base sequence and highly accurate nucleic acid subtraction.

## Description

### FIELD OF THE INVENTION

The present invention relates to the field of molecular biology, more specifically, it relates to a method for isolating or extracting a desired nucleic acid from a sample containing nucleic acids.

### BACKGROUND OF THE INVENTION

The isolation or extraction of a nucleic acid from a solution containing various nucleic acids is an important procedure in the field of genetic engineering for the preparation of various kinds of single-stranded DNAs or single-stranded DNA libraries, cloning of genes, or the like.

For example, there has been a method where various kinds of single-stranded DNAs or single-stranded DNA libraries are prepared by separating a certain nucleic acid from a solution containing various kinds of nucleic acids by using an immobilized single-stranded nucleic acid. Tsurui, Hiromichi (SAIBO-KOGAKU, Vol. 8, No. 7, 1989) proposed a method for the cloning of a desired gene, which comprises isolating one nucleic acid from a solution containing a mixture of nucleic acids by using a DNA probe immobilized on beads for high-pressure liquid chromatography (HPLC), attaching a primer DNA to a particular region of the single-stranded DNA, and converting the single-stranded DNA into double-stranded DNA by using DNA polymerase. This method, however, has problems, for example, a) only one kind of single-stranded DNA can be recovered at one time; and b) it is impossible to obtain a double-stranded DNA encompassing the entire region of the single-stranded DNA recovered, that is, only the region downstream from the site to which the primer attached can be doubled.

There is another method by which a single-stranded DNA library is prepared by introducing a double-stranded DNA fragments obtained by a known procedure such as an enzymatic treatment to M13 phage vector by using DNA ligase, transforming the vector into Escherichia coli, and recovering DNA in the form of M13 single-stranded DNA molecules. This method, however, has problems such as: a) only restricted kinds of vectors are available; b) the length of DNA fragments to be inserted into vectors is limited; and c) it is uncertain that all the DNAs can be transformed into E. coli.

Nucleic acid subtraction, by which a desired nucleic acid is obtained as the result of subtraction of undesired nucleic acids from a nucleic acid sample, is used to clone a desired gene specific for one of two relevant cell species. This happens when there are obtained two related cell species (e.g., T- and B-cells) from homologous organisms and only one species produce a desired protein; or there are a certain cell and a variant thereof and only one expresses a desired protein. The nucleic acid subtraction is carried out by isolating mRNA from one of two cell species, converting it into cDNA, hybridizing the cDNA to mRNA isolated from another cell species, recovering mRNA or cDNA which failed to hybridize, constructing a cDNA library by using the recovered mRNA or cDNA, and cloning a desired gene from said cDNA library.

The above-mentioned method has been carried out by contacting cDNAs immobilized non-specifically to membrane with a nucleic acid solution prepared from a desired cell under a condition for hybridization, removing mRNAs hybridized to immobilized cDNAs, recovering mRNA remaining in the solution as the specific mRNA for the desired cell. Alternatively, this can be performed by isolating a cDNA failed to hybridize to a complementary mRNA. This method, however, had problems such as: a) it takes a lot of time and labor to recover a cDNA from a cDNA library; b) as the cDNA is immobilized by the use of non-specific adsorption, the cDNA-mRNA hybridization is imperfect; and c) because of the low density of immobilized cDNA, not only troublesome operations are required but also a perfect reaction between cDNA and mRNA is hardly attained. Alternatively, a mRNA specific for a desired cell can be recovered by contacting mRNAs prepared from a desired cell with cDNAs for the subtraction, removing a double-stranded hybrid of mRNA and cDNA from the resultant solution by loading it onto hydroxyapatite column which adsorb exclusively double-stranded nucleic acids. This method, however, has problems such as: a) the use of column generally results in troublesome operations and diluted nucleic acid solution; b) the recovery rate of mRNA from column is extremely low; and c) it lacks the reproductivity.

As can be understood from the above, the existing methods for isolating or extracting nucleic acids have various problems and it has been demanded to develop an efficient method which solves these problems. The present inventors have studied extensively and found that a single-stranded nucleic acid immobilized at its terminus affords a highly accurate hybridization, i.e., a DNA-DNA or DNA-RNA hybrid, whereby makes it possible to recover a desired nucleic acid efficiently and completely. Accordingly, by the use of immobilized nucleic acids prepared by immobilizing two or more nucleic acids having different base sequences to separable insoluble supports at the terminus, it is possible not only to isolate and recover more than two nucleic acids simultaneously, but also to establish a highly-accurate and improved nucleic acid-subtracting method.

### DISCLOSURE OF THE INVENTION

Thus, the present invention provides a method for isolating or extracting a desired nucleic acid from a nucleic acid sample which comprises the following steps:
1) immobilizing a single-stranded nucleic acid at a terminus to an insoluble support;
2) contacting said immobilized single-stranded nucleic acid with said nucleic acid sample in a solution under a condition for nucleic acid hybridization; and
3) separating a nucleic acid hybridized to the single-stranded nucleic acid immobilized to said support from the sample.

As one embodiment, the present invention provides a method for isolating nucleic acids each having a complementary base sequence for each of immobilized nucleic acids from a sample all at once, which comprises immobilizing two or more kinds of single-stranded nucleic acids each having a different base sequence are immobilized to plural and separable supports, respectively, and bringing the immobilized nucleic acids to contact with a nucleic acid sample under a condition for hybridization.

A single-stranded nucleic acid in the nucleic acid sample solution, which hybridized to an immobilized single-stranded nucleic acid, can be recovered from the support by a conventional method such as heating, alkaline treatment and the like.

According to this method, for example, a double-stranded DNA encompassing the entire region including the desired DNA fragment can be obtained by converting a desired double-stranded DNA fragment in a nucleic acid sample solution into two single-stranded DNAs, hybridizing each single-stranded DNA with each of two kinds of immobilized-nucleic acids, recovering single-stranded DNAs, and annealing them.

Furthermore, a single-stranded DNA library consisting of those having base sequences specific for various kinds of vectors can be prepared by the use of immobilized DNAs which prepared by immobilizing DNAs having base sequences specific for each strand of various kinds of vector DNAs as a probe DNA. Therefore, the size of DNA fragments to be inserted into vectors is not limited. As a result, the possibility that all DNAs are transformed can be elevated when the DNA insertion and transformation are carried out as to several kinds of vectors by the present method.

As another embodiment, the present invention provides a method for extracting a specific nucleic acid from a sample by using immobilized single-stranded nucleic acids in the nucleic acid subtraction. Thus, a specific nucleic acid in a sample can be extracted by removing nucleic acids capable of hybridizing to immobilized single-stranded nucleic acids from said sample. This can be applied to the recovery of a nucleic acid (gene) responsible to and specific for a given disease such as cancer, which can be carried out by treating a mRNA mixture obtained from a variant cell such as cancer cell with immobilized cDNAs prepared from mRNAs originated from a normal cell and removing mRNAs having complementary sequences from the mixture to leave a specific mRNA. As can be understood by one of skilled in the art, the method illustrated above can be carried out by reacting immobilized cDNAs originated from a variant cell with a mRNA mixture obtained from a normal cell, obtaining an immobilized cDNA failed to hybridize, and cloning a gene encoding a protein specific for cancer cell.

For the method of the invention, preferably the immobilized single-stranded nucleic acid is a synthesized or reversely transcribed cDNA in vitro and the nucleic acid sample is a solution containing mRNA or cDNA.

Examples of insoluble supports usable in the present method are as follows.

### 1) Naturally-occurring membranes or synthetic membranous compounds

Examples include membranes (membranous materials) of natural or synthetic organic polymer such as nylon, nitrocellulose, polytetrafluoroethylene, polyethylene, polyisoprene, polystyrene membrane and the like. A membrane (e.g., nitrocellulose) which has a modified quality resulting from a chemical modification done on the surface of an organic polymer (e.g., cellulose) is also included. Furthermore, a derivative which is obtained by treating an existing membrane chemically and/or physically in such a manner as to facilitate the binding with nucleic acids or to make the handling easier is also included.

### 2) Naturally-occurring particles or particulate synthetic organic compounds

Examples include particles of natural or synthetic organic polymer such as nylon, nitrocellulose, polytetrafluoroethylene, polyethylene, polyisoprene, polystyrene and the like. Also included are a modified organic polymer particle obtained by a chemical treatment such as oxidation, reduction, hydrolysis or the like, or a physical treatment such as plasma irradiation or the like.

### 3) Membranous inorganic compounds

Examples include inorganic polymer membranes such as graphite, porous glass, silica membrane and the like; metallic membranes such as aluminium, apatite membrane and the like; ceramics membranes such as alumina, silicon nitrate membrane and the like; crystals such as salt and the like. Membranes having a modified quality obtained by chemically or physically treating the surface of those membranes are also usable.

### 4) Particulate inorganic compounds

Examples include inorganic polymer particles such as graphite, porous glass, silica and the like; metallic particles such as aluminium, apatite and the like; ceramics particles such as alumina and the like. Particles having modified quality obtained by a chemical or physical treatment (such as by ionplating) done on the surface of those mentioned above are also usable.

### 5) Materials having a gel-forming property such as polyacrylamide gel or agarose

When plural nucleic acids are desired to be isolated simultaneously according to the method of the present invention, plural supports selected from those mentioned above are used on the condition that they are separable. The separation of supports from each other can be carried out, for instance, as follows.
i) Separation can be carried out on the basis of the specific gravity by selecting supports having different specific gravity, performing the hybridization, adding a liquid having a specific gravity between these two supports, and separating heavy support from light support.
ii) Separation can be carried out on the basis of the difference in the shape by selecting supports of different and easily separable shape. Such combination of supports may be, for instance, plural membranous supports, plural kinds of particulate supports of different particle size, or membranous support(s) and particulate support(s).
iii) Separation by means of magnet can be carried out by selecting two kinds of supports, one of which can be recovered with a magnet (or magnetic force) and the other cannot.
iv) Any appropriate combination of the above-mentioned methods i), ii) and iii)
In the above methods, the term "plural kinds of supports" includes plural kinds of membranous supports made of the same material or particulate supports which are made of the same material but have a different particle size from each other.

Preferable combination of supports for the simultaneous separation of plural kinds of nucleic acids according to the method of the present invention is that of appropriate two particulate supports or two membranous supports, each support being selected from the group consisting of particulate organic compounds and organic and inorganic membranes. Taking the speed and efficiency of hybridization into consideration, a combination of appropriate two kinds of supports selected from organic and lnorganic particles are especially preferred. The separation of particulate supports can be conveniently carried out on the basis of the specific gravity.

Appropriate supports for the nucleic acid subtraction method of the present invention can be selected from those exemplified above.

For the subtraction method, particulate supports which can accelerate the hybridization and improve the efficiency are preferred, including particles of inorganic or organic compounds. An inorganic particle such as silica is especially preferred. Furthermore, silica for HPLC is most preferable because it has a less tendency to causing non-specific adsorption and can be easily dispersed and precipitated.

Immobilization of a single-stranded nucleic acid to a selected support is known in the art and includes a method which utilizes a non-specific adsorption due to the affinity between a single-stranded nucleic acid (hereinafter, a single-stranded nucleic acid to be immobilized is represented by a single-stranded DNA) and support, or a covalent bond formed between an amino group of said DNA and support a previously activated with ultraviolet radiation and the like. For obtaining an immobilized DNA to be used in the present method, said DNA being immobilized at its terminus, a DNA having one or more additional nucleotide molecules at its terminus, a DNA whose terminal nucleotide molecule being chemically modified, or a DNA having a functional group such as "Amino-link" (Applied Biosystems Inc. (ABI)) introduced at its terminus is used. Thus, such a single-stranded DNA is immobilized to a support via the additional nucleotide, chemically modified nucleotide, or a compound having functional group(s).

The immobilization will be hereinafter described in detail.

### A. Immobilization in which a single-stranded DNA is chemically modified at a terminus

1) A single-stranded DNA which has an introduced functional group suited for the immobilization, for instance, -NH₂, -COOH or the like, is prepared. When the DNA is synthesized, it can be carried out conventionally by means of a commercially available DNA synthesizer (Model 391 PCR-MATE) or the like.
   Introduction of a functional group can be, for example, performed as follows.
   a) Introduction of Amino-link 2 (ABI Inc.)
      A hexylamino group can be introduced to DNA at its terminus according to the following reaction scheme (Applied Biosystems Inc User Bulletin, No. 49, 1988). 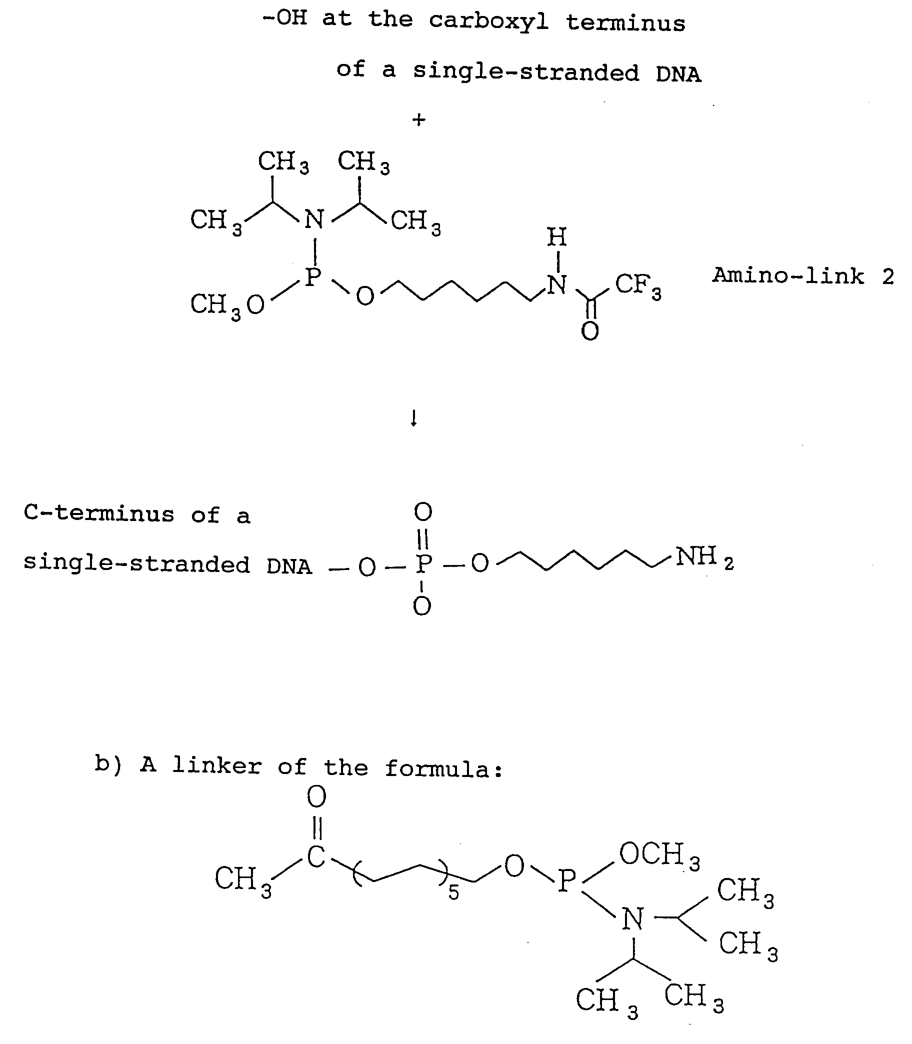 is introduced to a DNA at its terminus by using a DNA Synthesizer (e.g., ABI Inc., A-391 EP PCR-MATE), treating the linker portion to produce a functional group such as reactive aldehyde or carboxyl group at its terminus. It is then reacted, when the produced functional group is an aldehyde group, with a hydrazide compound of biotin to introduce biotin which can react with avidin to give a complex (see, Jonathan N. Kremsky et al., Nucleic Acid Research 1987, Vol. 15, from p. 2891).
   c) One to several tens of nucleotides having amino group(s) are added to a terminus of a complementary region of a DNA by means of a DNA Synthesizer.
   d) A base or a reactive derivative thereof suited for the binding to support is added to a terminus of a DNA with a terminal transferase (see, Deug G. and Wu R., Methods in Enzymology, Vol. 100, p. 96-116, 1983).
2) A complementary strand of the single-stranded DNA as obtained in 1) is then prepared by using a similar synthesizer and these are annealed to yield a double-stranded DNA.
3) The DNA prepared in 2) is bound to a insoluble support by mixing the DNA solution with a support for the immobilization. The way of immobilization varies depending on the chemical modifications applied to both of the DNA and support as illustrated below.
   a) A hydroxyl group (mainly, a diol group) on DNA or support is activated and reacted with mainly an amino group on support or DNA, wherein said activation can be carried out with trifluoroethanesulfonyl chloride (hereinafter, referred to as tresyl chloride) (K. Nilson and K. Mosbach, Biochem. Biophys. Res. Commun., 102, 449, 1981), CNBr (R. Axen et al., Nature, 214, 1302, 1967), trichlorotriazine (T.H. Finlay eta l., Anal. Biochem., 87, 77, 1978), epichlorohydrin (I. Matsumoto et al., J. Biochem., 85, 1091, 1979), bisoxylane (L. Sundberg and J. Porath, J. Chromatogr., 90, 87, 1974), divinyl sulfonic acid (J. Porath, Meth. Enzymol., 34, 27, 1974), benzoquinone (J. Brandt et al., Biochem. Biophys. Acta., 386, 196, 1975), carbonyldiimidazole (G.S.Bethell et al., J. Biol. Chem., 254, 2572, 1979), or the like.
   b) A carboxyl group (-COOH) on either of DNA or support is activated and bound to mainly an amino group (-NH₂) on support or DNA through condensation, wherein the activation can be carried out with a carbodiimide such as a water-soluble carbodiimide (A. Tengblad, Biochem. J., 199, 297, 1981; M. Funabashi et al., Anal. Biochem. 126, 414, 1982) or 2-ethoxy-1- ethoxycarbonyl-1,2-dihydroquinolin (EEDQ) (G. Saccomani et al., J. Biochem., 256, 12405, 1981; B. Belleuau and G. Malek, J. Am. Chem. Soc., 90, 1651, 1988).
   c) A desired DNA is linked with DNA ligase (linking enzyme) to an immobilized DNA which is bound conventionally to a support by a non-specific bond or at any sites other than a terminus.
   d) A DNA and a support are bound between hydrazide and aldehyde groups, or hydrazide and carboxyl groups on either of them. Hydrazide and aldehyde groups, upon mixing, form a hydrazone bond, which is converted into a covalent bond by reduction (Jonathan N. Kremsky et al., ibid). In case of a hydrazide and carboxyl groups, a carbodiimide is used for immobilization as noted in b) above.
   e) To DNA and support are introduced two different substances which have affinity to each other (e.g., biotin and avidin) and the former is immobilized to the latter by the use of the affinity (Jonathan N. Kremsky et al., ibid).
   f) After the activation of thiol groups on both of DNA and support, the former is immobilized to the latter (K. Brocklehurst et al., Biochm, J., 133, 573, 1973).
   g) DNA and support are bound via amino groups which exist on both of them by bromoacetamide method (P. Cuatrecasas, J. Biol. Chem., 245, 3059, 1970).
4) The immobilized double-stranded DNA as prepared above is denatured by heating (about 40°C or more) in a saline solution such as 2.4 M aqueous tetraethylammonium chloride solution, appropriately diluted 10 x SSC (1.5 M NaCl and 0.15 M sodium citrate, pH 7.0) or 0.1 - 2 M aqueous NaCl solution or alkali treatment. The solid phase is separated from a liquid phase by centrifugation to obtain an immobilized single-stranded DNA.

### B. Immobilization by the use of a double-stranded DNA with or without chemical modification at a terminus

Because a double-stranded DNA, in which either strand has one or more additional nucleotide molecules, can be bound to a support at its terminus as it is or after an appropriate chemical modification, it is useful for attaining the same purpose as noted above just by following the procedures 3) and 4) in method A. Such double-stranded DNAs can be prepared as follows.
a) A base or a reactive derivative thereof suited for the binding with a support is introduced to a terminus of only one strand of a double-stranded DNA.
b) A double-stranded DNA is cleaved with a restriction enzyme so as to generate a single strand at a terminus.
c) A DNA molecule having a functional group is ligated to a double-stranded DNA with a DNA ligase. For instance, a double-stranded DNA is digested with two restriction enzymes so as to generate a double-stranded DNA having different sequences in both terminae. To the resultant DNA is ligated a DNA having a functional group and being capable of specifically binding to one of cleaved ends with a DNA ligase to give a desired DNA having an functional group introduced at a terminus. In this case, the 5' terminus of the strand to be removed may be previously dephosphorylated. Thus, when a long double-stranded DNA having an extruded sequence at 5' terminus is prepared and dephosphoryulated with dephosphorylase followed by a cleavage to obtain a desired fragment having dephosphorylated 5' terminus.
d) A 3' terminal -OH group of a double-stranded DNA is activated by introducing a trichlorotriazine residue. The 5' terminal -OH group is exposed through the dephosphorylation of a double-stranded DNA as noted in c) above and activated with trichlorotriazine. As the -OH group in 5'-terminus is more active than that in 3'-terminus, the former can be activated specifically.

### C. Preparation of immobilized cDNA from mRNA

An oligomer (1 - 1000 bases) of deoxythymidine [hereinafter, referred to as oligo(dT)] is immobilized on a support in the same manner where a single-stranded DNA is immobilized to a support after a chemical modification at a terminus. To the oligo(dT) is hybridized a mRNA, and a cDNA is synthesized by using said mRNA as a template and oligo(dT) as a primer in the presence of a reverse transcriptase. The mRNA is removed conventionally to yield a cDNA immobilized to a support at its terminus via oligo(dT).

An immobilized single-stranded DNA suited for the method of the present invention can be obtained by any of the aforementioned methods A, B and C. In the working Examples below, immobilized single-stranded DNAs prepared by method A are used for the simultaneous isolation of plural nucleic acids and those prepared by method C for the nucleic acid subtraction. However, the present invention is never limited to a particular method for immobilization and can be carried out by using immobilized DNAs prepared by any methods other than those exemplified above.

Nucleic acid solutions suited as a nucleic sample are exemplified below:
1) a (native) double-stranded DNA extracted from a cell or a fragment thereof produced by a cleavage with a restriction enzyme or physical shearing force;
2) a naturally-occurring single-stranded DNA (including a denatured product of double-stranded DNA of 1) above by means of heat- or alkali-treatment);
3) a single- or a double-stranded DNA amplified in vitro by polymerase chain reaction (PCR) or the like and denatured product of said double-stranded DNA by heat- or alkali-treatment;
4) a synthetic DNA;
5) a naturally occurring RNA;
6) a synthetic RNA; and
7) a mixture containing any of DNA and RNA described in 1) to 5) above.

### BEST MODE FOR PRACTICING THE INVENTION

The simultaneous isolation of plural nucleic acids in a sample can be carried out as illustrated below.

Single-stranded nucleic acids having different base sequences are immobilized to plural kinds of separable supports to obtain "immobilized nucleic acids". The immobilized nucleic acids are then brought to contact with a sample solution containing nucleic acid mixture for the formation of hybrids between individual immobilized nucleic acid and corresponding single-stranded nucleic acid having a complementary base sequence in the sample.

The hybridization can be carried out by the use of any of known methods and reaction conditions. In this case, since the immobilized nucleic acid is fixed to a support at its terminus, no destructions of DNA bases due to the immobilization occur. Accordingly, the immobilized DNA can behave in the same manner as that in a liquid phase, and hence can hybridize to a nucleic acid (DNA, RNA) having a complementary base sequence efficiently and completely. Consequently, desired nucleic acids, each having complementary base sequence for corresponding immobilized single-stranded nucleic acid, can be isolated from the nucleic acid sample solution, respectively.

The desired single-stranded nucleic acid hybridized to an immobilized nucleic acid can be recovered as a single-stranded nucleic acid by separating the support and treating it by a conventional method such as heat- or alkali treatment.

The nucleic acid subtraction can be carried out as illustrated below.

Nucleic acids immobilized to insoluble supports at a terminus are contacted with a sample solution containing nucleic acids under a condition for hybridization. Nucleic acids having a complementary base sequence for either of the immobilized nucleic acids are removed and a nucleic acid remaining in the nucleic acid sample because of the lacking in complementary sequence is recovered. In this case, any known hybridization methods are again available. As mentioned above, the immobilized DNA is fixed at a terminus to a support and therefore no destructions in DNA bases due to the immobilization occur. Therefore, the immobilized nucleic acid can behave in similar of almost the same manner as that in liquid phase and hybridize to a corresponding nucleic acid (DNA, RNA) having a complementary base sequence efficiently and completely. Consequently, the desired nucleic acid having a complementary sequence can be removed from a nucleic acid sample.

According to this method, an immobilized cDNA can be synthesized on a support by using mRNA as a template, which in turn is used to extract merely a mRNA having complementary base sequence. For example, when there are two nucleic acid solutions each containing unknown mRNAs, a mRNA specific for only one solution can be recovered by preparing immobilized cDNAs by using mRNAs contained in one nucleic acid solution as a template and contacting them with another nucleic acid solution for the hybridization. The reverse of the above-mentioned procedures is also available. Alternatively, the same purpose can be achieved by analyzing a cDNA which failed to hybridize with any mRNAs.

Thus, according to this method, a specific nucleic acid which exists in either of two unidentified nucleic acid solutions can be recovered and cloned, and vice versa. Thus, the nucleic acid subtraction of the invention is useful in the genetic engineering, especially the gene cloning.

### Example 1

### Simultaneous Isolation of Plural Single-stranded DNAs in a Sample

### 1. Preparation of Immobilized Single-stranded DNA

(1) Four different DNAs having the following base sequences were prepared by using a DNA Synthesizer (391 EP PCR-MATE; Applied BI Inc.) and purified by HPLC.

These 25-base-sequences were selected from polycloning sites of pUC18.
1 X TGC AGG CAT GCA AGC TTG GCA CTG G
2 CCA GTG CCA AGC TTG CAT GCC TGC A
3 X CCA GTA CCA AGC TTG CAT GCC TGC A
4 TGC AGG CAT GCA AGC TTG GCA CTG G
Each base sequence is described in the form of a single strand and, from left to right, from 5' to 3' direction. The letter "X" represents the Amino-link 2 (ABI Inc.) which was introduced to the 5'-terminus at the final stage. The synthesis was carried out in 1 µM scale and the resultant DNA was purified by a known method and recovered in the range of 1.3 - 1.8 mg yield, respectively.

### 2. Immobilization

Immobilized nucleic acids wherein DNAs 1 and 3 are immobilized on each support at a terminus were prepared according to the following procedures.

### 1) Preparation of Immobilized DNA (a)

DNAs 1 and 2 (100 µg each) were evaporated to dryness. The DNA 1 (100 µg) was dissolved in 1 M NaCl (100 µl) and mixed with DNA 2 (100 µg). After warming the mixture for 2 min on a water bath (95 °C), it was cooled to 35°C over 1 hr. To the mixture is added 100 µl 0.4 M NaHCO₃ (pH 7.5).

To the mixture is added 20 mg tresyl-activated silica gel prepared as follows and reacted for 24 hr at room temperature. When the reaction completes, the silica gel was washed three times with 1 M NaCl and finally suspended in 1 ml 1 M NaCl. It was heated at 95°C and immediately centrifuged at 12000 rpm to remove the supernatant. These procedures were repeated 3 times to obtain an immobilized DNA (a) consisting of single-stranded DNA 1 immobilized to silica gel.

### 2) Preparation of Immobilized DNA (b)

DNAs 3 and 4 (100 µg each) were evaporated to dryness under vacuum. The DNA 3 (100 µg) was dissolved in 1 M NaCl (100 µl) and mixed with dried DNA 4 (100 µg). The mixture was warmed on a water bath (95°C) for 2 min and cooled to 35°C over 1 hr. To the resultant mixture was added 100 µl distilled water.

CM-5PW (30 mg, TOSO Inc.) was activated by heating at 4°C for 2 hr in a solution of 40 mg WSC (water-soluble carbodiimide: DOJIN Inc.) in 500 µl aqueous HCl (pH 4.0). The resultant gel was washed 3 times with distilled water, mixed with the nucleic acid solution as prepared above and reacted at 4°C for 5 hr with stirring. After heating for 5 min in a water bath (95°C), the mixture was immediately centrifuged at 12000 rpm to remove the supernatant, and the pellet was suspended in 0.5 M NaCl, heated at 95°C for 5 min and centrifuged at 12000 rpm, which treatments were repeated 2 more times, to give an immobilized DNA (b) consisting of single-stranded DNA 3 immobilized to CM-5PW.

### 2. Preparation of Tresyl-activated Silica Gel

The following procedures were carried out while avoiding the contamination with water by the use of an appropriate apparatus such as dry box, sterile pack filled with dry nitrogen or the like. The term "silica gel" may be hereinafter abbreviated to "gel".
1) Acetone and pyridine are previously dehydrated by means of molecular sieve.
2) One ml of dehydrated acetone, 100 µl pyridine, and a small stirrer-chip are placed in a 10 ml measuring flask.
3) One gram of gel is washed quickly with acetone, dehydrated acetone while aspirating on a glass filter (# 5 mesh) and promptly put into the measuring flask prepared in 2) above.
4) To the gel is added dropwise 100 to 200 µl tresyl chloride (Fluka Inc.) over about 1 min with a vigorous stirring by the stirrer while keeping the flask at a temperature around 0°C with, for example, an ice-packed plastic bag.
5) The measuring flask is capped and the reaction is continued for 20 min at about 0°C at a lowered stirring speed to prevent the gel from being broken.
6) When the reaction completes, the gel is transferred onto a glass filter and washed with acetone, acetone/5 mM HCl (1:1), and 5 mM HCl.
7) The gel is further washed with acetone and the solvent is evaporated thoroughly on the filter.
8) The gel is transferred into an egg-plant type flask and acetone is removed under vacuum. The gel, because tresyl is unstable at a high temperature, is dried under a low temperature by means of an ice-packed plastic bag as used in 4) above or the like to yield a tresyl-activated silica gel.

### 3. Preparation of Nucleic Acid Sample

A nucleic acid sample was prepared by using 100 µg of commercially-available pUC18 DNA (Takara Shuzo Inc.) according to the following procedures and hybridized to immobilized DNAs (a) and (b).
1) A DNA is cleaved in a solution comprising:

| | |
|---|---|
| pUC18 (Takara Shuzo Inc.) (500 µg/ml) | 200 µl |
| EcoRI (Takara Shuzo Inc.) (20 units/ml) | 20 µl |
| EcoRI buffer (Takara Shuzo Inc.) (x10) | 50 µl |
| Sterilized distilled-water | 230 µl |
| Total | 500 µl. |

The mixture is reacted for 1 hr at 37°C and the reaction is stopped by the addition of 10 µl 0.5 M ethylenediaminetetraacetic acid (EDTA; pH8.0).
2) The reaction solution of 1) above is combined with 50 µl 5M sodium acetate and 1 ml ethanol and allowed to stand for 20 min at -80 °C.
3) It is centrifuged at 12000 rpm, 4°C for 10 min.
4) The supernatant is removed and the precipitates are washed with 70% ethanol cooled at -20°C, centrifuged at 12000 rpm, 4°C for 30 sec and the supernatant is removed.
5) The precipitates are dried in a desiccator.
6) The precipitates are dissolved thoroughly in 200 µl 2.4 M tetraethylammonium chloride (TEACL; Wako Junyaku Inc.) and allowed to stand for 5 min at 75°C.
7) To the precipitates of 6) are added all of the both immobilized DNAs (a) and (b) as prepared in 2. above, and the mixture is stirred well and allowed to stand for 15 min at 20°C.
8) The mixture of 7) is centrifuged at 12000 rpm and the supernatant is transferred to another eppendorf tube.
9) To the mixture in the tube is added 200 µl 2.4 M TEACL, stirred thoroughly and centrifuged at 12000 rpm for several seconds to pellet the gel, and the supernatant is removed.
10) The pellet is mixed with 200 µl 2.4 M TEACL and the mixture is combined with 300 µl tetrabromoethane/chloroform adjusted to the specific gravity of 1.5 and centrifuged at 6000 rpm for several seconds.
11) Separated gels (each support) are respectively recovered.
12) Each gel is suspended in 2.4 M TEACL (100 µl).
13) The gel is maintained in as solution at 70°C for 10 min to allow the hybridized DNA to dissolve in the liquid phase, which (sample DNA solution) is then recovered.

### 4. Assay of the Recovered DNA

The single-stranded DNA recovered from a sample solution containing DNA mixture in 3. above was assayed according to the following procedures.
1) A piece of filter paper (5.5 x 5.5 cm) is moistened in 10 ml 20 x SSPE.solution. The 20 x SSPE solution is prepared by dissolving 174 g NaCl, 31.2 g NaH₂PO₄ 2H₂O, and 7.4 g EDTA 2Na 2H₂O in 800 ml water, adjusting the pH to 7.4 with NaOH and the volume to 1 liter, and sterilizing in an autoclave.
2) The filter paper prepared in 1) above is placed in a 96-well Miniholed Apparatus (Biodot: Bio-Rad Inc.).
3) A 5 x 5 cm BIODYNE membrane (PALL Inc.) is moistened in sterilized water, soaked in 20 x SSPE, and attached to the Miniholed Apparatus (Biodot: Bio-Rad Inc.).
4) All wells are washed once with 20 x SSPE.
5) Each sample DNA solution (5 µl each) is added to two wells of the Miniholed.
6) The DNA is adsorbed to a membrane with suction.
7) The membrane is took off and washed twice with 20 x SSPE.
8) The membrane is dried at 80°C under vacuum and cut into 2 (2 spots).
Each of two pieces of membrane is independently subjected to the following treatments.
9) The membrane is soaked into 2 x SSPE.
10) The membrane is then soaked in 0.5 ml hybridization solution and allowed to stand for 2 hr at 65°C. The hybridization solution consists of the following ingredients:

| | |
|---|---|
| 20 x SSPE | 15 ml |
| 50 x Denhardt's solution | 2 ml |
| 10% SDS (sodium dodecyl sulfate) | 2.5 ml |
| 10 µg/ml salmon sperm DNA | 0.5 ml |
| sterilized water | 30 ml |
| total | 50 ml. |

11) Liquid phase is discarded and 12.5 ml of freshly prepared hybridization solution is added. To the solution is added 50 µl each of ³²P-labeled DNA probes (A) and (B) and reacted overnight at 65 °C.
12) The membrane is washed four times with 65°C 0.2 x SSPE and 0.1% SDS for 15 min.
13) The membrane is placed on a vinylidene chloride film.
14) An X-ray film is inserted between them and is exposed overnight at -80°C.
15) The film is developed to detect positive or negative result.

The assay results for each of two DNA fractions indicate that DNAs each having a complementary base sequence for individual immobilized DNA are recovered. The results are shown in Table 1 below.

**Table 1**

| | Probe A | Probe B |
|---|---|---|
| DNA in silica gel fraction | + | - |
| DNA in CM-5PW fraction | - | + |
| Note: + ; positive; and - ; negative | | |

### 5. Preparation of DNA Probe

DNA probes (A) and (B) were prepared according to the following procedures.
1) The following DNAs 5 and 6 are synthesized by a DNA Synthesizer (ABI Inc., 391 EP PCR-MATE) and purified following the instructions provided by ABI Inc.
5 TGC AGG CAT GCA AGC TTG GCA CTG G
6 CCA GTG CCA AGC TTG CAT GCC TGC A
Each base sequence is described, from left to right, from 5' to 3' direction. DNA 5 and 6 were labeled to yield DNA probes (A) and (B), respectively.
2) Each of purified DNAs 5 and 6 is dissolved in sterilized water and diluted to 10 pmol/µl with distilled water on the basis of the measurement of absorbance at 260 nm in a spectrophotometer.
3) Each DNA 5 and 6 is labeled with γ-³²P at its 5'-terminus in a solution containing the following ingredients:

| | |
|---|---|
| DNA (10 pmol/ml) | 1.0 µl |
| 10 x bacteriophage T4 polynucleotide kinase buffer* | 2.0 µl |
| [γ-³²P]ATP (sp. act. 5000 Ci/mmol; 10 mCi/ml aqueous solution) (10 pmol) | 5.0 µ |
| water | 11.0 µl. |

| | |
|---|---|
| *: 10 x bacteriophage T4 polynucleotide kinase | |

buffer consists of the following ingredients:

| | |
|---|---|
| Tris-HCl (pH 7.6) | 0.5 M |
| MgCl₂ | 0.1 M |
| dithiothreitol (DTT) | 50 mM |
| spermidine HCl | 1 mM |
| EDTA (ph 8.0) | 1 mM. |

4) After the addition of 8 units bacteriophage T4 polynucleotide kinase, the mixture is allowed to stand for 45 min at 37°C.
5) The mixture is heated at 68°C for 10 min to stop the reaction.
6) The mixture is combined with 40 µl sterilized water and mix thoroughly.
7) A 240 µl of 5M sodium acetate is added to it and mixed thoroughly.
8) To the mixture is added 750 µl ethanol (- 20 °C) and mixed thoroughly.
9) The mixture is centrifuged at 12000 rpm, 0°C for 20 min.
10) After the addition of 500 µl 80% ethanol, the mixture is stirred, centrifuged at 12000 rpm, 0°C for 1 min and the supernatant is discarded.
11) The precipitate is dissolved well into 100 µl TE buffer (pH 7.6) which consists of 10 mM Tris-HCl (pH 7.5) and 1 mM EDTA.

The specific radio activity was 540 Ci/mmol on a liquid scintillation counter.

These results obtained in Example 1 demonstrate that the present method is effective for the preparation of various kinds of single-stranded nucleic acids at a time, indicating that the present invention is useful for the preparation of a DNA sequence sample for Sanger's method which requires a single-stranded DNA.

Additionally, a single-stranded DNA library, which had been able to be be prepared only in M13 phage system prior to the present invention, is now can be prepared easily irrespective of the length of a DNA to be inserted. A single-stranded DNA library can be prepared even from a double-stranded DNA library.

Furthermore, contrary to the existing cloning method which uses an immobilized probe, the present method makes it possible to clone a fragment encompassing the entire region of a desired DNA (nucleic acid). Thus, according to the present method, the entire region of a desired fragment can be cloned in high yield by recovering each strand of a desired double-stranded DNA by the use of two kinds of separable and immobilized DNA probes, and annealing them.

### Example 2

### Nucleic Acid Subtraction

### 1. Preparation of Tresyl-activated Silica Gel

Tresyl-activated silica gel was prepared in the same manner as noted in Example 1.

### 2. Synthesis of Oligo(dT) and Immobilization to Supports

A single-stranded DNA having the following base sequence was prepared by using a DNA Synthesizer (ABI Inc.) in 1 µM scale, and purified to obtain 1.5 mg DNA.
5' X TTT TTT TTT TTT TTT TTT TTT TTT TTT 3'
- X:: Amino-link 2 (ABI Inc.)
The resultant DNA (27-mer dT) was purified conventionally by high performance liquid chromatography. A portion (500 µg) of said DNA was dried under vacuum. A portion (100 µg) of this dried DNA was dissolved in 1 M NaCl (100 µl) and 100 µl 0.4 M NaHCO₃ (pH 7.5) was added thereto. To the solution was added tresyl-activated silica gel and reacted for 24 hr at room temperature. After the reaction, silica gel was washed thrice with 1 M NaCl and suspended in 1 ml 1 M NaCl. After heating for 5 min at 95°C, the mixture was promptly centrifuged at 12000 rpm, and the supernatant was discarded to give a 27-mer oligo(dT) immobilized to silica gel as the residue.

### 3. Preparation of mRNA

1) Liver (2 g) and spleen (2 g) were removed from a 3-week-old BALB/c mouse and each was used separately for the preparation of mRNA as follows.
2) The tissue is homogenized in 70 ml 5.5 M guanidine thiocyanate solution (hereinafter, referred to as 5.5 M GTC solution) in a polytetrafluoroethylene glass homogenizer. The 5.5 M GTC solution is prepared by adjusting a mixture containing 5.5 M GTC, 25 mM sodium citrate (Wako Junyaku) and 0.5% Sarkosyl to pH 7.0 with NaOH and filtering. The solution is, just before the use, adjusted to 0.2 M 2-mercaptoethanol.
3) DNA is mechanically cleaved by pipetting the homogenate with a disposable syringe equipped with a #18 needle.
4) A half volume of CsTFA-0.1 M EDTA solution is placed in an autoclaved centrifuging tube (Polyaromer Inc.) and the sample of 3) is layered on it, which was followed by the centrifugation at 23000 rpm, 15°C for 24 hr. CsTFA-0.1 M EDTA solution was prepared by mixing CsTFA (Pharmacia Inc.) and 0.25 M EDTA (pH 8.0) and adjusting the ρ to 1.51 with distilled water.
5) After the removal of protein and DNA layers with a pipet, liquid is discarded to obtain the residue. The residue is dissolved in 4 ml 4 M GTC solution prepared by diluting 5.5 M GTC with distilled water.
6) The insoluble materials are removed by centrifuging at 10000 rpm for 10 min.
7) To the supernatant is added 100 µl 1 M acetic acid and 3 ml ethanol and the mixture is allowed to stand for 5 hr at -20 °C.
8) The mixture is centrifuged at 10000 rpm for 20 min.
9) The residue is dissolved in 3 ml TE and centrifuged at 10000 rpm for 10 min and the supernatant is recovered. The TE buffer consists of the following ingredients:

| | |
|---|---|
| Tris-HCl (pH 7.5) | 10 mM |
| EDTA | 1 mM. |

10) After the addition of 0.3 ml 2 M NaCl and 6 ml ethanol, the mixture is allowed to stand for 3 hr at -20 °C.
11) The mixture is centrifuged at 10,000 rpm for 10 min to recover the precipitates.
12) The precipitates are dissolved in a buffer A consisting of 10 mM Tris-HCl (pH 7.5), 0.5 M NaCl, 1 mM EDTA and 0.1 % SDS to the concentration of 0.5 mg/ml.
13) The solution of 12) is mixed with an equal amount of 1 M NaCl.
14) The mixture is treated at 65°C for 5 min and cooled quickly.
15) The insoluble materials are removed by centrifuging at 12,000 rpm for 5 min.
16) A column packed with 0.5 g Type 7 oligo(dT) cellulose (Pharmacia Inc.) previously swollen with distilled water is washed with 5 ml 0.1 M NaOH and then equilibrated with buffer A. This is followed by the equilibration with 10 ml 0.5 M NaCl.
17) The supernatant of 15) is passed through the column of 16).
18) The eluate is again passed through the same column.
19) The column is developed with 10 ml buffer A. Fractions (500 µl each) are collected, and each fraction is assayed by the measurement of A 260, and positive peaks are recovered (fractions 4 - 7).
20) After the addition of 250 ml 3 M sodium acetate, pH 5.1 (Wako Junyaku Inc.) and 6 ml ethanol, the mixture is stirred thoroughly and kept at - 20 °C for precipitation.

According to the above-mentioned procedures, desired mRNAs from kidney and spleen of 3-week-old BALB/c mouse are prepared, respectively.

### 4. Synthesis of Immobilized cDNA and Nucleic Acid Substraction with It

A cDNA immobilized on a support was synthesized by using mRNA isolated from a liver of 3-week-old BALB/c mouse as a template. A mRNA-containing nucleic acid solution prepared from spleen of a 3-week-old BALB/c mouse was contacted with the immobilized cDNA for the hybridization and analyzed for a mRNA(s) remaining in the solution.
1) A 5 µg mRNA extracted from liver of a 3-week-old BALB/c mouse was dissolved in 10 µl H₂O.
2) To the solution is added 1 µl 100 mM CH₃HgOH and the mixture is allowed to stand for 10 min.
3) After the addition of 2 µl 700 mM 2-mercaptoethanol (20-fold dilution of a raw material), the mixture is stirred thoroughly to inactivate CH₃HgOH.
4) It is followed by an immediate addition of 60 units (6 µl) RNase Inhibitor (TAKARA Shuzo Inc.) and the mixture is stirred and allowed to stand for 5 min.
5) To the mixture is added 10 mg of 27-mer oligo(dT)-immobilized gel prepared in 2. above.
6) A mixture consisting of the following ingredients are reacted at 45°C for 2 hr:

| | |
|---|---|
| soluble component (liquid phase) of mixture of 5) above | 12 µl |
| first-strand-synthetic solution | 10 µl |
| 80 mM Na-pyrophosphate | 2.5 µl |
| dNTPs mixture | 5 µl |
| H₂O | 14.5 µl |
| reverse transcriptase (Seikagaku Kogyo Inc.) | 1000 units. |

The first-strand-synthetic solution consists of the following ingredients:

| | |
|---|---|
| Tris-HCl (pH 8.3) | 250 mM |
| MgCl₂ | 50 mM |
| KCl | 250 mM. |

7) To the reaction solution of 6) above is added 2 units RNase H (Behringer Manheim, Yamanouchi Inc.) and reacted at 12 °C for 60 min, and at 22°C for 60 min.
8) The enzyme is inactivated at 70°C for 10 min.
9) The mixture is centrifuged at 12000 for 1 min to remove the supernatant.
10) The precipitate is washed with 500 µl TE and centrifuged at 120000 rpm for 1 min to remove the supernatant.
11) The precipitatse are suspended in 500 µl TE.
12) To the mixture is added 100 µl 2 mg/ml (dA)₃₀ (a 30-mer of adenine).
13) After the addition of 100 µl 5 M NaCl, the mixture is stirred and allowed to stand for 15 min at 37°C.
14) The supernatant is removed after the centrifugation at 12000 rpm for 1 min.
15) The precipitates are combined with mRNA solution which is prepared by dissolving 5 µg mRNAs originated from spleen in 100 µl buffer A, heating at 70 °C and cooling.
16) To the mixture is added 10 µl 5 M NaCl.
17) The volume is adjusted to 500 µl with water.
18) The reaction is carried out at 55 °C for 10 min with stirring.
19) The mixture is centrifuged at 12000 rpm for 10 min.
20) The supernatant is recovered.
21) After the addition of 1.5 ml ethanol, the mixture is allowed to stand for 5 hr at - 20°C.
22) The mixture is centrifuged at 12000 rpm for 10 min, cooled at 4°C and the supernatant is discarded.
23) The precipitate is dissolved in 5 µl TE.
24) When a mixture of 1 µl of the solution of 23) and 20 µl 1 µg/ml ethidium bromide was irradiated with UV light, an orange color was developed.

This result demonstrates that a RNA(s) exists in the solution obtained in 23), which indicates that spleen contains a mRNA(s) which does not hybridize to cDNAs prepared from mRNAs originated from liver.

The result obtained in Example 2 shows that the nucleic subtraction method of the invention makes it possible to recover a specific nucleic acid which does not exists commonly in two unknown nucleic acid mixtures. For instance, when nucleic acids derived from a normal cell are immobilized and used to treat a nucleic acid mixture obtained from a target cell concerning a certain disease (e.g., cancer cell) according to the method of the present invention, the recovered nucleic acid can be assigned to be a gene specific for said disease.

Thus, the subtraction of the present invention can be a useful measure not only for the elucidation of a gene(s) specific for a disease, but also for the efficient isolation of various genes like those responsible to aging and specific to organs by selecting an appropriate combinations of nucleic acid solutions therefor.

## Claims

1. A method for isolating or extracting a desired nucleic acid from a nucleic acid sample which comprises:
1) immobilizing a single-stranded nucleic acid at a terminus to an insoluble support;
2) contacting said immobilized single-stranded nucleic acid with said nucleic acid sample under a condition for nucleic acid hybridization; and
3) separating a nucleic acid hybridized to the single-stranded nucleic acid immobilized to the support from the sample.

2. The method as claimed in Claim 1, wherein the immobilized single-stranded DNA is obtained by immobilizing two or more kinds of single-stranded nucleic acids each having a different base sequence to plural and separable supports, respectively, and the resultant immobilized single-stranded DNAs are contacted with a nucleic acid sample and two or more nucleic acids each hybridizing to each of the immobilized single-stranded nucleic acids are isolated from the nucleic acid sample all at once.

3. The method as claimed in Claim 1, wherein nucleic acids hybridizing to the immobilized single-stranded nucleic acids are removed and a nucleic acid remaining in the solution is recovered.

4. The method as claimed in Claim 1, wherein the single-stranded nucleic acid to be immobilized to a support is a synthetic DNA.

5. The method as claimed in Claim 1, wherein the immobilized single-stranded nucleic acid is prepared by immobilizing a double-stranded DNA consisting of a single-stranded DNA modified at a terminus and a complementary strand therefor to a support via said modified terminus and removing the complementary strand.

6. The method as claimed in Claim 1, wherein the immobilized single-stranded nucleic acid is an immobilized cDNA which is prepared by the following steps:
1) immobilizing an oligomer of deoxythymine to a support;
2) hybridizing a mRNA to said immobilized oligomer deoxythymine;
3) synthesizing a cDNA by using said mRNA as a template and said oligomer deoxythymidine as a primer with a reverse transcriptase; and
4) removing said mRNA.

7. The method as claimed in Claim 1, wherein the nucleic acid solution is a solution which contains at least one kind of nucleic acid selected from the group consisting of mRNA and cDNA.
